# EUROPEAN PATENT SPECIFICATION

(11) **EP 3 768 358 B1**
(45) Date of publication and mention of the grant of the patent: **28.10.2026**
(21) Application number: 19709969.0
(22) Date of filing: 15.03.2019
(51) Int. Cl.: A61M 5/24, A61M 5/28, A61M 5/32

(54) **A SHELL FOR RECEIVING AN INJECTION SYSTEM COMPRISING A SYRINGE RECEIVED IN A SAFETY DEVICE**
SCHALE ZUR AUFNAHME EINES INJEKTIONSSYSTEMS MIT EINER IN EINER SICHERHEITSVORRICHTUNG AUFGENOMMENE SPRITZE
ENVELOPPE DESTINÉE À RECEVOIR UN SYSTÈME D'INJECTION COMPRENANT UNE SERINGUE REÇUE DANS UN DISPOSITIF DE SÉCURITÉ

(30) Priority: 19.03.2018 EP 18162487
(43) Date of publication of application: 27.01.2021
(73) Proprietor: Becton Dickinson France, 38800 Le Pont-de-Claix (FR)
(72) Inventor: CARREL, Franck, 38220 Saint Jean De Vaulx (FR); VALENTIN, Stéphane, 38470 L'Albenc (FR); MUNSCH, Stephane, 38450 Vif (FR)
(74) Representative: Germain Maureau
(86) International application number: PCT/EP2019/056583
(87) International publication number: WO 2019/179895

(56) References cited:
- EP-B1- 1 474 194
- WO-A1-2013/159059
- WO-A1-99/17823
- US-A1- 2018 008 776

## Description

The present invention relates to a shell for receiving an injection system comprising a syringe received in a safety device. The invention further relates to a safety system for a syringe which comprises a safety device and such a shell, and to a syringe system comprising a syringe and such a safety system.

Some illnesses, such as multiple sclerosis or arthritis, necessitate that a drug be injected into a patient regularly, such as every day or every week. The drug is available under the form of prefilled drug delivery devices, such as prefilled syringes.

Usually, the patient is trained to self injection and is capable to proceed to the injection of the drug by himself. Nevertheless, injection systems are provided to make injection easier for the patient.

EP 1474194 discloses a safety support device for a syringe and an assembly comprising one such device and a syringe. WO 2013/159059 discloses an anti-needle stick safety device for an injection device. US 2018/008776 discloses a syringe holder and a pharmaceutical liquid administration set.

Although known injections systems are generally satisfactory, they do not always meet all of the user's expectations. In particular, they may be fairly difficult to use for some patients, for example those having deformed hands. Moreover, while injections systems aim at reducing the patient's apprehension, the needle may still be visible, which may frighten some patients.

There is therefore a need for an improved injection system that would make self injection even easier, safer, and less difficult emotionally.

According to a first aspect, the invention relates to a shell for receiving an injection system comprising a syringe received in a safety device, the safety device having a body configured to receive the syringe, and a shield movable relative to the body and capable of covering at least part of a syringe needle, at least one finger flange extending outwardly from the body or the safety shield. The shell is according to claim 1.

In this application, the distal end of a component or apparatus must be understood as meaning the end furthest from the hand of the user and the proximal end must be understood as meaning the end closest to the hand of the user, with reference to the injection device intended to be used with said component or apparatus. As such, in this application, the distal direction must be understood as the direction of injection with reference to the injection device, and the proximal direction is the opposite direction.

Thus, owing to the invention, the injection system is housed in an outer shell which is easier to manipulate, even for a patient with deformed hands. Indeed, as the shell is configured to receive the injection system, it has larger dimensions than said injection system, and is therefore easier to grab and to handle. Furthermore, as the shell is an outer component which is not directly into contact with the syringe, contrary to the safety system, there are fewer limitations on its shape, which can be appropriately designed to be easier to handle.

Another advantage of the shell according to the invention is that it makes it possible to hide the syringe needle so as to lower the patient's apprehension. Indeed, generally, the safety device is required to be transparent to enable visual inspection of the syringe received in the safety device, and of the syringe content. This means that, even if the syringe needle does not extend beyond the safety device distal end, said needle can be seen through the safety device. With the invention, the injection system can be inserted into the shell after visual inspection, which means that the shell is not required to be transparent. Providing an outer opaque shell makes it possible to hide the needle from the patient's view without preventing the preliminary visual inspection step.

Another significant advantage of the invention is that the injection system is very satisfactorily maintained in the shell. More specifically, owing to the snap features and protrusion(s), the shell is configured:
- on the one hand, to improve the retention of the safety device inside the shell by limiting or deleting the gap between the finger flange and the shell, which prevents the safety device from shaking inside the shell;
- and, on the other hand, to achieve such a high-quality retention effect without requiring a high insertion force for inserting the injection system inside the shell, and without deforming or otherwise altering the safety device. Any risk of creating losable particles of the constitutive material of the safety device or shell is therefore avoided.

More specifically, the snap features can be used to secure the finger flange in the mounted position, i.e. to secure axially the safety device inside the shell and prevent it from being uncoupled from the shell. As regards the protrusion, its aim is to tighten the safety device against the shell, preferably substantially orthogonally to the axis, substantially without exerting a compression force likely to damage the safety device.

Because the snap features possibly the protrusion can flex outwardly, for example from a rest position when the injection system is not received in the shell, the injection system insertion is facilitated. In particular, the injection system can be inserted until the finger flanges are located distally from the snap features, and axially facing the protrusion.

In an embodiment, the shell is made as one single piece. For example, the shell can be made by injection of a plastic material, such as ABS (acrylonitrile butadiene styrene) or a copolymer ABS - PC (polycarbonate). Alternatively, the casing, snap features and protrusion(s) can be made as one single piece.

The snap feature may have a distal face, preferably orthogonal to the shell axis. This distal face may be configured to form an abutment for securing the finger flange in the mounted position. The snap feature may further have a proximal face, preferably a sloped proximal face for facilitating insertion of the safety device in the shell.

The protrusion may have a holding face configured to push the finger flange inwardly. The holding face may be located distally from the snap feature proximal face. The holding face may extend along a direction secant to the snap feature distal face.

The protrusion may further have a proximal face, preferably a sloped proximal face for facilitating insertion of the safety device in the shell. However, the proximal face of the protrusion is not necessarily sloped; in particular this is not necessary if the proximal face of the snap feature is located proximally from the proximal face of the protrusion and is sloped.

Said tab can extend longitudinally.

One wall of the casing may comprise a slot which substantially has the shape of a U having two legs extending longitudinally and a base orthogonal to the shell axis, the area of the casing wall located inside the slot forming said tab. The base may be located at the distal part of the slot. The line joining the ends of the legs not connected to the base is preferably located at the proximal part of the slot. Said line may form a hinge allowing the tab to be deformed or to pivot outwardly.

The protrusion can extend distally from the snap feature and from the tab, partly in the slot base.

According to an embodiment, the shell can comprise two protrusions each arranged on one lateral wall of the casing, the protrusions being opposed relative to the axis along a lateral direction. Said lateral wall is the portion of the peripheral wall of the casing which is parallel to the lateral direction along which the finger flanges of the safety device shield extend.

In other words, if a transverse plane is defined as being orthogonal to said lateral direction and containing the shell longitudinal axis, the casing may comprise two half-casings located on both sides of said transverse plane, and the shell can comprise at least one protrusion on one lateral wall of each half-casing.

If only one protrusion is provided for a given half-casing, said protrusion pushes the finger flange against the opposite lateral wall of the half-casing.

Alternatively, the shell may comprise one protrusion on each one of the opposed lateral walls of the casing, for example on each one of the opposed lateral walls of each half-casing, i.e. four protrusions. With such a configuration, the protrusions allow tightening the finger flange between them, which helps balancing the safety system.

According to another embodiment, the shell can comprise one protrusion on one transverse wall of each half-casing, i.e. two protrusions. Said transverse wall is the portion of the peripheral wall of the casing which is orthogonal to the lateral direction along which the finger flanges of the safety device shield extend. Therefore, the protrusions are in contact with the lateral ends of the finger flanges.

The casing may form an enlarged portion of the shell with respect to the housing, said enlarged portion extending in a lateral direction. Such a configuration may help improving the easy handling of the shell. In particular, a user may place his/her fingers on the distal face of said enlarged portion.

The shell may further comprise a distal end provided with an inner compartment configured to receive a cap remover. The cap remover can be used to remove a cap mounted on the syringe distal end for covering the needle before pricking.

The shell may further comprise at least four, and preferably six, ribs inside the housing, the ribs being configured to centre the safety in the shell.

The shell may further comprise at least one insertion stop, preferably two insertion stops, configured to form a distal abutment for the injection system in the mounted position. The insertion stop may be formed by an extension portion of one rib in the casing, configured so that the finger flange can abut on said insertion stop in the mounted position.

According to a second aspect, the invention relates to a safety system for a syringe, comprising:
- a safety device having a body configured to receive the syringe, and a shield movable relative to the body and capable of covering a syringe needle, the shield having finger flanges which extend laterally adjacent to a proximal end of the shield;
- a shell as previously described, the safety device being mounted in the shell, the finger flanges of the safety device shield being maintained relative to the shell by the snap feature and protrusion.

According to a third aspect, the invention relates to a syringe system comprising a safety system as previously described, and a syringe inserted in the body of the safety device, the syringe comprising a needle.

The shell may have a length such that, in the mounted position, the needle exceeds the distal end of the shell of a pre-determined length. The shell may have an adjustable length such that, in the mounted position, the needle length exposure from the distal end of the shell can be controlled. In practice, depending on the requirements, such as the pharmaceutical company requirements or the medical staff requirements, the maximum pricking depth in the patient's skin may vary. The shell length is chosen accordingly to obtain the required and predetermined needle length beyond the shell distal end. According to a non limiting example, the needle may extend beyond the shell distal end by a distance in the range of 0,4 to 0,7 mm.

According to another aspect, the invention relates to a shell for receiving an injection system including a syringe received in a safety device. The safety device includes a body configured to receive the syringe, a shield movable relative to the body and configured to cover at least part of a syringe needle, and at least one finger flange extending outwardly from the body or the safety shield. The shell extends along a longitudinal axis and includes a housing which is substantially tubular and configured to receive at least a distal portion of the safety device and a casing configured to receive the finger flange of the safety device. The casing includes at least two snap features configured to flex outwardly during insertion of the finger flange in the casing so that the finger flange is insertable distally from a distal face of the snap feature, and a protrusion located distally from each snap feature distal face. The protrusion is configured to exert an inward biasing force on the finger flange once the finger flange is inserted distally from the distal face of the snap feature.

According to another aspect, the invention relates to a safety system for a syringe, including a safety device having a body configured to receive the syringe, and a shield movable relative to the body and configured to cover a syringe needle, the shield having finger flanges which extend laterally adjacent to a proximal end of the shield. The safety system further includes a shell as described herein, the safety device mounted in the shell, the finger flanges of the safety device shield maintained relative to the shell by the snap feature and the protrusion.

According to another aspect, the invention relates to a syringe system including a safety device having a body configured to receive the syringe, and a shield movable relative to the body and configured to cover a syringe needle, the shield having finger flanges which extend laterally adjacent to a proximal end of the shield. The safety system further includes a shell as described herein, the safety device mounted in the shell, the finger flanges of the safety device shield maintained relative to the shell by the snap feature and the protrusion. The syringe system further includes a syringe inserted in the body of the safety device, the syringe comprising a needle.

These and other features and advantages will become apparent upon reading the following description in view of the figures attached hereto representing, as a non-limiting example, an embodiment of the invention.
Figure 1 shows an embodiment of a syringe system according to the invention;
Figure 2 is an exploded view of an injection system which can be inserted in a shell according to the invention to form a syringe system;
Figure 3 shows the injection system before use;
Figure 4 shows the injection system once injection is completed and the system has been removed from the injection site;
Figure 5 is an enlarged view of detail A of Figure 1;
Figure 6 is a perspective top view of the syringe system of Figure 1;
Figure 7 is a detailed view of the shell showing a snap feature and a protrusion;
Figure 8 is a cross section of the safety system showing the protrusions tightening one finger flange of the safety device shield;
Figure 9 is a perspective partial view of the safety system;
Figure 10 is a cross section of the safety system along plane P9 of figure 9;

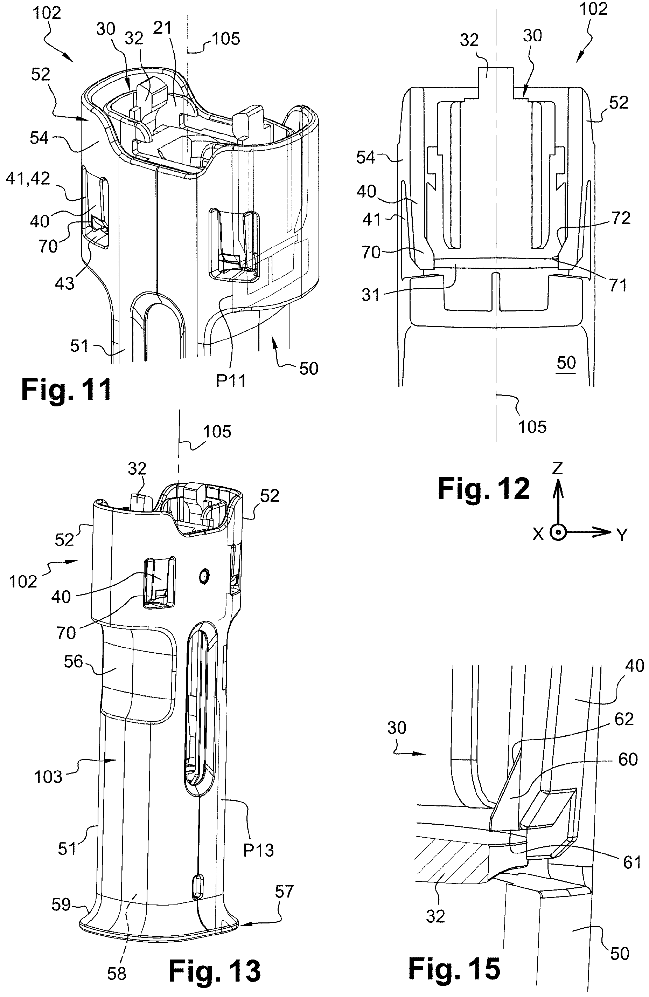

Figure 11 is a perspective partial view of the safety system;
Figure 12 is a cross section of the safety system along plane P11 of figure 11;
Figure 13 is a perspective view of the safety system;
Figure 14 is a cross section of the safety system along plane P13 of figure 13;
Figure 15 an enlarged view of detail B of Figure 14;
Figures 16a-16c schematically illustrates successive configurations of the syringe system during insertion of the injection system into the shell;
Figure 17 is a cross section of the syringe system, showing centring ribs;
Figure 18 is another cross section of the syringe system, showing an insertion stop.

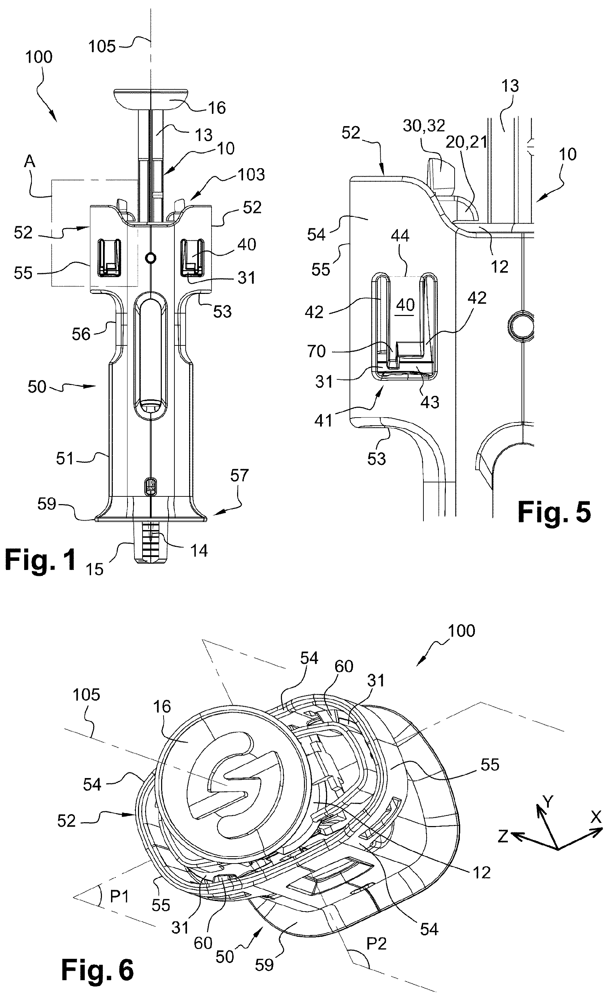

Figure 1 shows a syringe system 100 which comprises an injection system 101 and a shell 50 for receiving the injection system 100.

The injection system 101 includes a syringe 10 and a safety device 103.

The syringe 10 comprises a barrel 11 - preferably having a proximal outer flange 12 - a plunger rod 13 and a needle 14. A cap 15 may further be provided to cover the needle 14 before use, to avoid injuries and contamination. The cap 15 may be removably mounted on the proximal end of the barrel 11. The plunger rod 13 may further comprise a proximal member 16 pushed by a user's finger to perform injection, which can be disc-shaped.

The safety device 103 is configured to receive the syringe 10. The safety device 103 comprises:
- a body 20 configured to receive the syringe 10;
- a shield 30 movable relative to the body 20 and capable of covering at least part of the syringe needle 14;
- finger flanges 31 which extend laterally adjacent to a proximal end of the shield 30 or to a proximal end of the body 20. The finger flanges 31 have a distal face 34.

The shell 50 and safety device 103 form a safety system 102 for the syringe 10. The syringe system 100 comprises the safety system 102 and the syringe 10.

As shown on figures 1 and 6, the shell extends along an axis 105. Preferably, the syringe system 100 and its constitutive parts have the same axis 105. Longitudinal direction Z is defined as the direction of axis 105. The terms "distally" and "proximally" are used with reference to said longitudinal direction Z. Lateral direction X is defined as a direction orthogonal to axis 105 and as being the general direction along which the finger flanges 31 of the shield 30 extend. The transverse direction Y is defined as the direction orthogonal to the longitudinal direction Z and to the lateral direction X.

The syringe system 100, or at least part of its constitutive parts, may have one or two axes of symmetry, namely a lateral plane P1 parallel to (X,Z) and/or a transverse plane P2 parallel to (Y,Z).

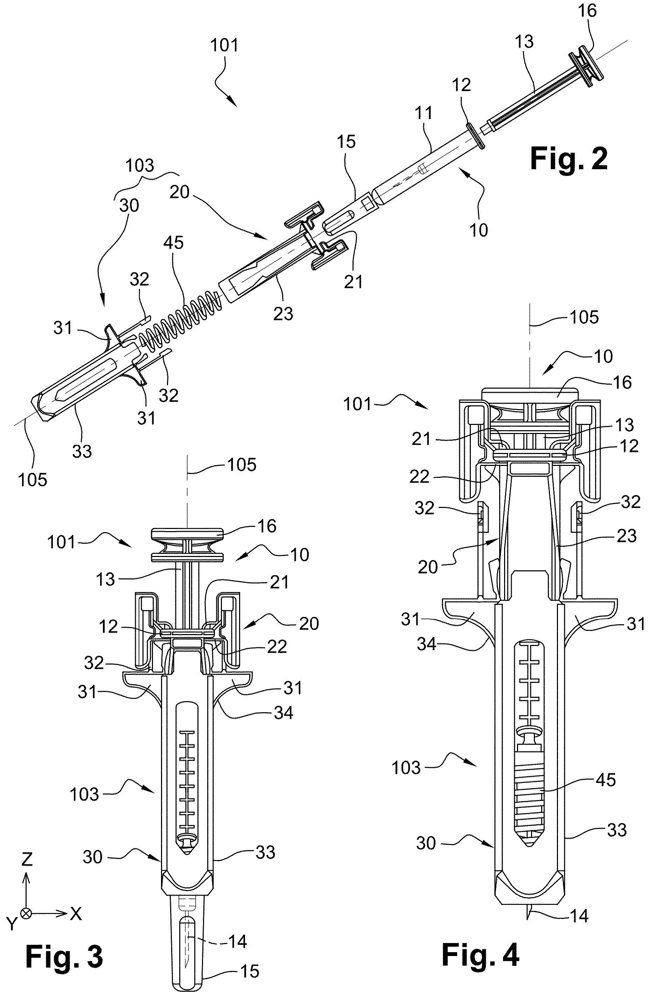

A non limiting example of an injection system 101 is illustrated in figures 2, 3 and 4, and can be at least partially similar to the system described in WO 2013/159059.

The syringe 10 is received in a tubular portion 23 of the body 20 and is secured in the mounted position by appropriate means, such as fasteners 21 projecting inwardly at the proximal part of the body 20. The flange 12 of the barrel 11 may be snapped between said fasteners and a wall 22 of the body 20 which extends orthogonally to axis 105 and which is located distally from the fasteners 21.

The shield 30 preferably comprises a tubular portion 33 which receives the body 20. The shield 30 can move axially relative to the body 20. Before use, as shown in figure 3, the body 20 is maintained in position relative to the shield 30, against the action of a spring 45, for example by means of at least one clamp 32 which can cooperate with the fasteners 21 or another part of the body 20 (see figure 5).

In an embodiment, as shown in figure 3, the needle 14 may extend beyond the distal end of the safety device 103 before use. When injection has been completed, the clamp 32 or other retaining member is released. This can be achieved by the action of the plunger rod 13, for example of the proximal member 16, which can cause the fasteners 21 to be freed from the clamps 32.

As a result, when the injection system 101 has been removed from the patient's skin, the spring 45 causes the shield 30 to move distally relative to the body 20, thereby covering at least part of the needle 14, as shown in figure 4.

In another embodiment (not shown), the needle 14 may be covered by the shield 30 before use of the injection system 101. Thus, during pricking, the shield 30 moves proximally relative to the body against the actin of the spring 45, so that the needle 14 can extend beyond the shield distal end. After injection has been completed and when the injection system 101 has been removed from the patient's skin, the spring 45 causes the shield 30 to move distally relative to the body 20, thereby covering at least part of the needle 14.

The shell 50 comprises a housing 51 which is substantially tubular and configured to receive a distal portion of the safety device 103. The shell 50 further comprises a casing 52 configured to receive the finger flange 31 of the safety device.

The casing 52 can form an enlarged portion of the shell 50 with respect to the housing 51, said enlarged portion extending in the lateral direction X. The casing can be located at the proximal part of the shell 50.

The casing 52 may comprise two half-casings located on both sides of transverse plane P2.

On each side of transverse plane P2, the casing 52 can have a distal face 53, preferably substantially orthogonal to axis 105, and a peripheral wall including two opposed lateral walls 54, preferably extending parallel to P1 and one transverse wall 55, preferably extending parallel to P2. As can be seen in figure 6, the junctions between one lateral wall 54 and the transverse wall 55 is curved, the casing 52 thus having a substantially U-shaped cross section. The proximal end of the casing 52 is open.

In other words, the shell 50 can substantially have the shape of a T, when viewed along the transverse direction Y. This makes handling of the shell 50 easier, as the user can place his/her fingers on the distal face 53 of the casing 52.

Moreover, the housing 51 may have two opposed lateral recesses 56 located distally from the casing 52, so that grasping the shell 50 is made even easier for the user.

The distal end 57 of the shell 50 may be provided with an inner compartment 58 configured to receive a cap remover (not shown), and/or with an outer collar 59 allowing improving the proper positioning of the syringe system 100 on the injection site.

The casing 52 comprises:
- at least two snap features 60 configured to secure the finger flange 31 of the safety device shield 30 in the mounted position; the snap feature 60 may act as an axial abutment which prevents the safety device 103 from moving proximally relative to the shell 50 and ultimately from being uncoupled from the shell 50;
- and a protrusion 70 associated with each snap feature. Each protrusion 70 projects inwardly. Each protrusion is configured to tighten the safety device 103 against the shell 50, so as to prevent - or at least limit - shaking of the safety device 103 inside the shell 50. In the mounted position, the protrusions can push the finger flange of the safety device towards or against the shell for limiting or preventing a movement of the safety device relative to the shell.

In the exemplary illustrated embodiment, the shell 50 comprises one set of one snap feature 60 and one protrusion 70 on each one of the opposed lateral walls 54 of the casing 52, i.e. four of such sets. However, other implementations could be envisaged provided they make it possible to provide satisfactory holding of the safety device 103 inside the shell 50.

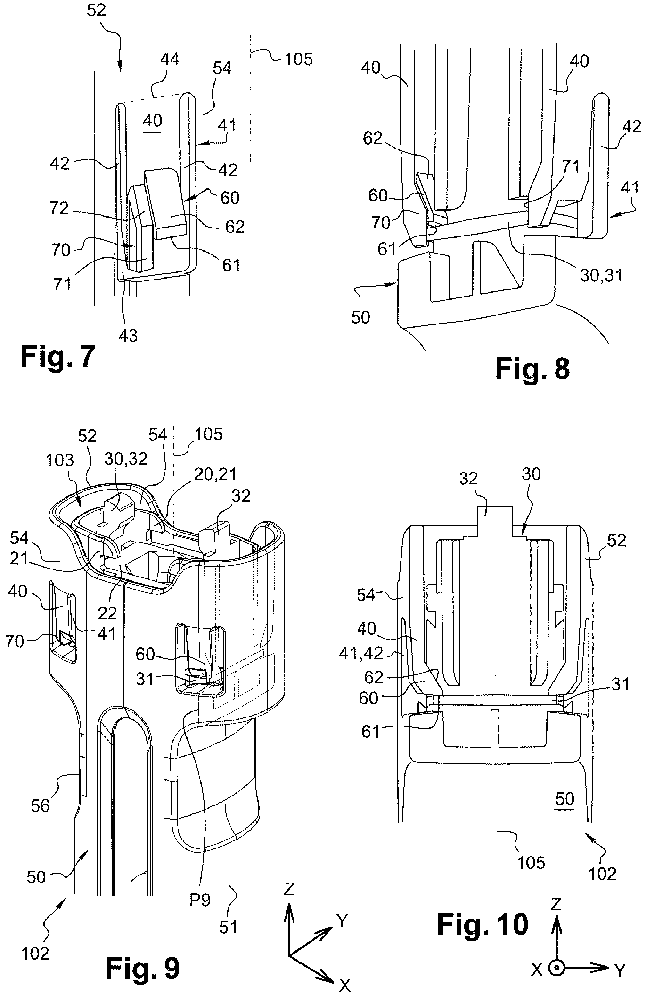

One snap feature 60 and one protrusion 70 can be arranged inwardly on one and the same tab 40. As shown on figures 5 and 7, each lateral wall 54 of the casing 52 comprises a slot 41 which substantially has the shape of a U having two legs 42 extending longitudinally and a base 43 orthogonal to the shell axis 105. The area of the casing wall 54 located inside the slot 41 forms said tab 40. The tab 40 can be substantially rectangular and can extend longitudinally.

The base 43 of the slot 41 may be located at the distal part of the slot 41, while the line 44 joining the ends of the legs 42 which are not connected to the base 43 may preferably be located at the proximal part of the slot 41. Said line 44 may form a hinge allowing the tab 40 to be deformed or to pivot outwardly.

As seen in figure 7, the snap feature 60 can have a distal face 61 preferably orthogonal to the shell axis 105, which can be configured to form an abutment for securing the finger flange 31 in the mounted position. The snap feature 60 can further have a proximal face 62, preferably sloped for facilitating insertion of the safety device 103 in the shell 50.

The protrusion 70 can have a holding face 71, preferably substantially longitudinal in a rest position or in the mounted position of the safety device 103 in the shell 50. The holding face 71 may be configured to push the finger flange 31 inwardly. The holding face 71 may be located distally from the distal face 61. The protrusion 70 can have a proximal face 72, preferably sloped for facilitating insertion of the safety device 103 in the shell 50.

The holding face 71 can be configured so as to increase the gripping effect on the finger flange 31. To that end, the holding face 71 may have a specific surface condition (such as micro-reliefs), or may be covered by a thin film of an appropriate material, such as a thermoplastic elastomer (TPE) or a thermoplastic polyurethane (TPU). An increased gripping effect makes it possible to further reduce shaking between the safety device 103 and the shell 50.

The protrusion 70 may extend distally from the snap feature 60, and preferably also distally from the tab, partly in the slot base 43.

One casing 52 and corresponding snap feature(s) 60, protrusion(s) 70 and possible tab(s) 40 can be made as one single piece. Preferably, the whole shell 50 can be made as one single piece. The process used can be injection of a plastic material, such as ABS (acrylonitrile butadiene styrene) or a copolymer ABS - PC (polycarbonate).

In practice, the syringe 10, preferably prefilled, is mounted in the safety device 103 to form the injection system 101. Then, the injection system 101 is inserted into the shell 50 up to the mounted position.

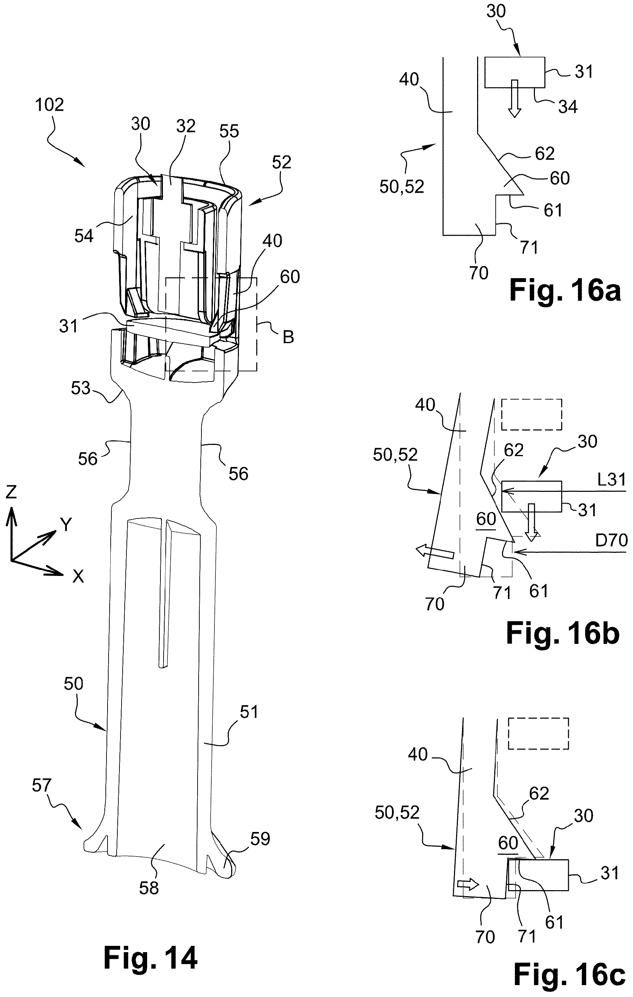

During this insertion movement, as schematically shown in figures 16a to 16c, the finger flanges 31 of the safety device shield 30 move distally relative to the shell 50, inside the casings 52.

Initially, the tab 40, snap feature 60 and protrusion 70 are in the rest position, as illustrated in figure 16a.

Then, as shown in figure 16b, the distal face 34 of the finger flange 31 comes into contact with the proximal face 62 of the snap feature 60, causing the tab 40 to elastically flex outwardly. This allows insertion of the finger flanges 31 distally from the distal face 61 of the snap feature 60.

When the injection system 101 is the mounted position, i.e. at the end of the insertion movement into the shell 50, the finger flange 31 is located distally from the distal face of the snap feature 62, and secured in the mounted position owing to the distal face 61 of the snap feature 60, as shown in figure 16c.

Moreover, as the distance D70 between the holding faces 71 of the protrusions 70, along direction Y, at rest, is smaller than the transverse dimension L31 of the finger flanges 31 (see figure 16b), when the injection system 101 is the mounted position, the protrusions 70 exert an inward biasing force on the finger flanges 31. In other words, each of the facing protrusions 70 pushes the finger flange 31 of the safety device shield 30 towards the other protrusion 70. The finger flange 31 is thus tightened between the protrusions 70, which limits or even prevents a movement of the safety device 103 relative to the shell 50.

The protrusions 70 act as anti-vibration dampers for preventing the safety device 103 from shaking inside the shell 50.

Thanks to the outward flexibility of the tab 40, the snap feature 60 is also flexed outwardly during insertion of the injection system 101. As a consequence, the length of the snap feature 60 - orthogonally to the tab 40, i.e. along the transverse direction Y in the exemplary embodiment - can be fairly great without hampering the insertion movement, in particular when the finger flange 31 comes into contact with the snap feature 60. Such a greater length of the snap feature 60 is advantageous as it provides a more efficient abutment for retaining the safety device 103 snapped inside the shell 50.

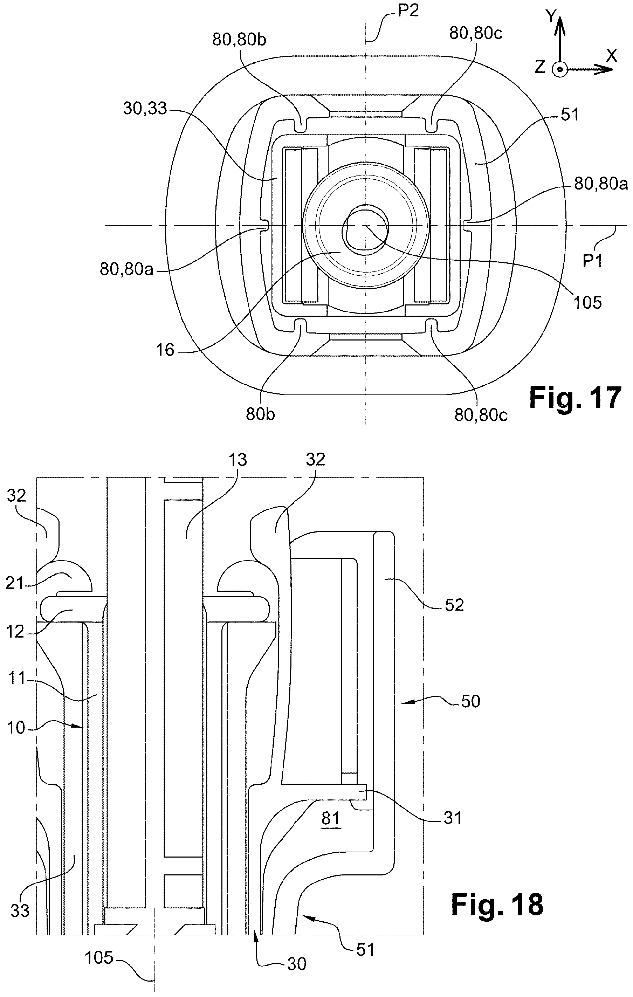

Advantageously, the shell 50 may further comprise ribs 80 for centring the safety device 103 in the shell 50, preferably both during insertion and in the mounted position. These ribs 80 can be seen in figure 17, which is a cross section of the syringe system 100 in a plane orthogonal to axis 105, at the level of the lateral recesses 56 of the housing 51.

The ribs 80 may extend over part or all of the inner face of the housing 50. The ribs 80 may project orthogonally inwardly from the inner face of the housing 50. They may contact or be located very close to the outer face of the tubular portion 33 of the shield 30.

The shell 50 may comprise at least four ribs 80, and preferably six ribs 80, namely: two facing ribs 80a arranged on opposed transverse walls of the housing 50; and two sets of facing ribs 80b, 80c arranged on opposed lateral walls of the housing 50.

Besides, the shell 50 can be provided with an insertion stop 81 configured to form an abutment for the injection system 101 in the mounted position, and to prevent said injection system 101 to further move distally relative to the shell 50 from said mounted position. The insertion stop 81 also allows controlling the appropriate mounted position. For that purpose, the insertion stop 81 can be adjusted or changed.

There may be provided two insertion stops 81, preferably arranged opposite to one another relative to axis 105.

As illustrated in figure 18, the insertion stop 81 can project inwardly from the distal face 53 of the casing 52, and can be configured to cooperate with the finger flanges 31. The insertion stop 81 may be formed in the continuity of one rib 80a, in plane P1.

In the mounted position, as shown in figure 1, the needle 14 can extend beyond the distal end 57 of the shell 50 of a pre determined length. Preferably, in order to prevent needle injury, the needle is covered by the cap 15, which cap 15 can be covered by a cap remover as previously described. Once the cap 15 has been removed, the syringe system 100 is ready for use.

By providing an outer shell, the invention makes the injection system 101 easier and less traumatic to use. By additionally preventing the safety device 103 from shaking inside the shell 50, the invention ensures that the safety device 103 is not damaged by its movements, and limits or prevents noises related to the shaking, which is detrimental to the perceived quality of the syringe system 100.

The invention is of course not limited to the embodiments described above as examples, but encompasses all technical alternatives of the means described as well as combinations thereof.

## Claims

1. A shell (50) for receiving an injection system (101) comprising a syringe (10) received in a safety device (103), the safety device (103) having:
- a body (20) configured to receive the syringe (10);
- a shield (30) movable relative to the body (20) and capable of covering at least part of a syringe needle (14);
- at least one finger flange (31) extending outwardly from the body (20) or the safety shield (30);
the shell (50) extending along a longitudinal axis (105) and comprising:
- a housing (51) which is substantially tubular and configured to receive at least a distal portion of the safety device (103);
- and a casing (52) configured to receive the finger flange (31) of the safety device (103);
wherein the casing (52) comprises:
- at least two snap features (60) configured to flex outwardly during insertion of the finger flange (31) in the casing (52) so that the finger flange (31) may be inserted distally from a distal face (61) of the snap feature (60);
- a protrusion (70) located distally from each snap feature distal face (61), the protrusion (70) being configured to exert an inward biasing force on the finger flange (31) once the finger flange (31) is inserted distally from the distal face (61) of the snap feature (60),
wherein each protrusion (70) protrudes distally from one snap feature (60), thereby forming a single tab (40) with the snap feature (60), the tab (40) being configured:
- to elastically flex outwardly during insertion of the safety device (103) in the shell (50), to allow insertion of the finger flanges (31) distally from the distal face (61) of the snap feature (60);
**characterized in that** the tab (40) is configured - to exert an inward biasing force on the finger flange (31) when the finger flange (31) is located distally from the distal face (61) of the snap feature (60),
whereby the protrusion (70) is configured to tighten the safety device (103) against the shell (50) substantially orthogonally to the axis (105), a gap between the finger flange (31) and the shell (50) being deleted, so that the safety device (103) is prevented from shaking inside the shell (50).

2. The shell according to claim 1, **characterized in that** the shell (50) is made as one single piece.

3. The shell according to claim 1 or claim 2, **characterized in that** the protrusion (70) has a holding face (71) configured to push the finger flange (31) inwardly, the holding face (71) extending along a direction secant to the snap feature distal face (61).

4. The shell according to any one of claims 1 to 3, **characterized in that** the snap feature (60) further comprises a sloped proximal face (62) for facilitating insertion of the safety device (103) in the shell (50).

5. The shell according to any one of claims 1 to 4, **characterized in that** the protrusion (70) has a sloped proximal face (72) for facilitating insertion of the safety device (103) in the shell (50).

6. The shell according to any one of claims 1 to 5, **characterized in that** one wall (54) of the casing (52) comprises a slot (41) which substantially has the shape of a U having two legs (42) extending longitudinally and a base (43) orthogonal to the shell axis (105), the area of the casing (52) wall located inside the slot forming said tab (40).

7. The shell according to any one of claims 1 to 6, **characterized in that** it comprises at least two protrusions (70) each arranged on one lateral wall (54) of the casing (52), the protrusions (70) being opposed relative to the axis (105) along a lateral direction (X).

8. The shell according to any one of claims 1 to 7, **characterized in that** it comprises one protrusion (70) on each one of the opposed lateral walls (54) of the casing (52).

9. The shell according to one of claims 1 to 8, **characterized in that** the casing (52) forms an enlarged portion of the shell (50) with respect to the housing (51), said enlarged portion extending in a lateral direction (X).

10. The shell according to any one of claims 1 to 9, **characterized in that** it further comprises a distal end (57) provided with an inner compartment (58) configured to receive a cap remover.

11. The shell according to any of claims 1 to 10, further comprising at least four ribs (80) inside the housing (51), the ribs (80) being configured to centre the safety device (103) in the shell (50).

12. A safety system (102) for a syringe (10), comprising:
- a safety device (103) having a body (20) configured to receive the syringe (10), and a shield (30) movable relative to the body (20) and capable of covering a syringe needle (14), the shield (30) having finger flanges (31) which extend laterally adjacent to a proximal end of the shield (30);
- a shell (50) according to any one of the preceding claims, the safety device (103) being mounted in the shell (50), the finger flanges (31) of the safety device shield (30) being maintained relative to the shell (50) by the snap feature (60) and protrusion (70).

13. A syringe system (100) comprising a safety system (102) according to claim 12 and a syringe (10) inserted in the body (20) of the safety device (103), the syringe (10) comprising a needle (14).

14. The syringe system according to claim 13, **characterized in that** the shell (50) has a length such that, in the mounted position, the needle (14) exceeds the distal end (57) of the shell (50) of a pre determined length.

## Patentansprüche

1. Hülle (50) zum Aufnehmen eines Injektionssystems (101), umfassend eine in einer Sicherheitsvorrichtung (103) aufgenommene Spritze (10), wobei die Sicherheitsvorrichtung (103) aufweist:
- einen Körper (20), der konfiguriert ist, um die Spritze (10) aufzunehmen;
- ein Schild (30), das in Bezug zum Körper (20) beweglich ist und zum Abdecken mindestens eines Teils einer Spritzennadel (14) imstande ist;
- mindestens eine Fingerauflage (31), die sich vom Körper (20) oder dem Sicherheitsschild (30) nach außen erstreckt;
wobei sich die Hülle (50) entlang einer Längsachse (105) erstreckt und umfasst:
- ein Gehäuse (51), das im Wesentlichen röhrenförmig ist und konfiguriert ist, um mindestens einen distalen Abschnitt der Sicherheitsvorrichtung (103) aufzunehmen;
- und eine Verkleidung (52), die konfiguriert ist, um die Fingerauflage (31) der Sicherheitsvorrichtung (103) aufzunehmen;
wobei die Verkleidung (52) umfasst:
- mindestens zwei Schnappmerkmale (60), die konfiguriert sind, um sich beim Einführen der Fingerauflage (31) in die Verkleidung (52) nach außen zu biegen, sodass die Fingerauflage (31) distal von einer distalen Fläche (61) des Schnappmerkmals (60) eingeführt werden kann;
- einen Vorsprung (70), der sich distal von jeder distalen Fläche (61) des Schnappmerkmals befindet, wobei der Vorsprung (70) konfiguriert ist, um eine nach innen gerichtete Vorspannkraft auf die Fingerauflage (31) auszuüben, sobald die Fingerauflage (31) distal von der distalen Fläche (61) des Schnappmerkmals (60) eingeführt wird,
wobei jede Vorsprung (70) distal von einem Schnappmerkmal (60) hervorsteht, wodurch eine einzelne Lasche (40) mit dem Schnappmerkmal (60) gebildet wird, wobei die Lasche (40) konfiguriert ist:
- um sich beim Einführen der Sicherheitsvorrichtung (103) in die Hülle (50) elastisch nach außen biegen, um ein Einführen der Fingerauflagen (31) distal von der distalen Fläche (61) des Schnappmerkmals (60) zu ermöglichen;
**dadurch gekennzeichnet, dass** die Lasche (40) konfiguriert ist, um eine nach innen gerichtete Vorspannkraft auf die Fingerauflage (31) auszuüben, wenn sich die Fingerauflage (31) distal von der distalen Fläche (61) des Schnappmerkmals (60) befindet,
wobei der Vorsprung (70) konfiguriert ist, um die Sicherheitsvorrichtung (103) im Wesentlichen orthogonal zur Achse (105) an der Hülle (50) festzuziehen, wobei ein Spalt zwischen der Fingerauflage (31) und der Hülle (50) beseitigt wird, sodass ein Wackeln der Sicherheitsvorrichtung (103) in der Hülle (50) verhindert wird.

2. Hülle nach Anspruch 1, **dadurch gekennzeichnet, dass** die Hülle (50) als ein einziges Stück gefertigt ist.

3. Hülle nach Anspruch 1 oder Anspruch 2, **dadurch gekennzeichnet, dass** der Vorsprung (70) eine Haltefläche (71) aufweist, die konfiguriert ist, um die Fingerauflage (31) nach innen zu drücken, wobei sich die Haltefläche (71) entlang einer Sekantenrichtung zur distalen Fläche (61) des Schnappmerkmals erstreckt.

4. Hülle nach einem der Ansprüche 1 bis 3, **dadurch gekennzeichnet, dass** das Schnappmerkmal (60) weiter eine geneigte proximale Fläche (62) zum Erleichtern einer Einführung der Sicherheitsvorrichtung (103) in die Hülle (50) umfasst.

5. Hülle nach einem der Ansprüche 1 bis 4, **dadurch gekennzeichnet, dass** der Vorsprung (70) eine geneigte proximale Fläche (72) zum Erleichtern einer Einführung der Sicherheitsvorrichtung (103) in die Hülle (50) aufweist.

6. Hülle nach einem der Ansprüche 1 bis 5, **dadurch gekennzeichnet, dass** eine Wand (54) der Verkleidung (52) einen Schlitz (41) umfasst, der im Wesentlichen die Form eines U aufweist, das zwei sich längs erstreckende Schenkel (42) und eine zur Hüllenachse (105) orthogonale Basis (43) aufweist, wobei sich der Bereich der Wand der Verkleidung (52) innerhalb des Schlitzes befindet, der die Lasche (40) bildet.

7. Hülle nach einem der Ansprüche 1 bis 6, **dadurch gekennzeichnet, dass** sie mindestens zwei Vorsprünge (70) umfasst, die jeweils an einer Seitenwand (54) der Verkleidung (52) angeordnet sind, wobei die Vorsprünge (70) in Bezug zur Achse (105) entlang einer seitlichen Richtung (X) gegenüberliegen.

8. Hülle nach einem der Ansprüche 1 bis 7, **dadurch gekennzeichnet, dass** sie jeweils einen Vorsprung (70) an jeder der gegenüberliegenden Seitenwände (54) der Verkleidung (52) umfasst.

9. Hülle nach einem der Ansprüche 1 bis 8, **dadurch gekennzeichnet, dass** die Verkleidung (52) einen in Bezug zum Gehäuse (51) vergrößerten Abschnitt der Hülle (50) bildet, wobei sich der vergrößerte Abschnitt in einer lateralen Richtung (X) erstreckt.

10. Hülle nach einem der Ansprüche 1 bis 9, **dadurch gekennzeichnet, dass** sie weiter ein distales Ende (57) umfasst, das mit einem inneren Fach (58) bereitgestellt ist, das konfiguriert ist, um einen Kappenentferner aufzunehmen.

11. Hülle nach einem der Ansprüche 1 bis 10, weiter mindestens vier Rippen (80) im Inneren des Gehäuses (51) umfassend, wobei die Rippen (80) konfiguriert sind, um die Sicherheitsvorrichtung (103) in der Hülle (50) zu zentrieren.

12. Sicherheitssystem (102) für eine Spritze (10), umfassend:
- eine Sicherheitsvorrichtung (103), die einen Körper (20) aufweist, der konfiguriert ist, um die Spritze (10) aufzunehmen, und einen Schild (30), der in Bezug zum Körper (20) beweglich ist und zum Abdecken einer Spritzennadel (14) imstande ist, wobei der Schild (30) Fingerauflagen (31) aufweist, die sich seitlich an ein proximales Ende des Schilds (30) angrenzend erstrecken;
- eine Hülle (50) nach einem der vorstehenden Ansprüche, wobei die Sicherheitsvorrichtung (103) in der Hülle (50) montiert ist, wobei die Fingerauflagen (31) des Schilds (30) der Sicherheitsvorrichtung durch das Schnappmerkmal (60) und den Vorsprung (70) in Bezug zur Hülle (50) gehalten werden.

13. Spritzensystem (100), das ein Sicherheitssystem (102) nach Anspruch 12 und eine in den Körper (20) der Sicherheitsvorrichtung (103) eingeführte Spritze (10) umfasst, wobei die Spritze (10) eine Nadel (14) umfasst.

14. Spritzensystem nach Anspruch 13, **dadurch gekennzeichnet, dass** die Hülle (50) eine Länge aufweist, sodass die Nadel (14) in der montierten Position das distale Ende (57) der Hülle (50) um eine vorbestimmte Länge übersteigt.

## Revendications

1. Enveloppe (50) pour recevoir un système d'injection (101) comprenant une seringue (10) reçue dans un dispositif de sécurité (103), le dispositif de sécurité (103) ayant :
- un corps (20) configuré pour recevoir la seringue (10) ;
- un protecteur (30) mobile par rapport au corps (20) et capable de couvrir au moins une partie d'une aiguille de seringue (14) ;
- au moins une bride de doigt (31) s'étendant vers l'extérieur depuis le corps (20) ou le protecteur de sécurité (30) ;
l'enveloppe (50) s'étendant le long d'un axe longitudinal (105) et comprenant :
- un logement (51) qui est sensiblement tubulaire et configuré pour recevoir au moins une partie distale du dispositif de sécurité (103) ;
- et un boîtier (52) configuré pour recevoir la bride de doigt (31) du dispositif de sécurité (103) ;
dans laquelle le boîtier (52) comprend :
- au moins deux éléments d'encliquetage (60) configurés pour fléchir vers l'extérieur lors de l'insertion de la bride de doigt (31) dans le boîtier (52), de sorte que la bride de doigt (31) puisse être insérée distalement depuis une face distale (61) de l'élément d'encliquetage (60) ;
- une saillie (70) située distalement par rapport à chaque face distale (61) d'élément d'encliquetage, la saillie (70) étant configurée pour exercer une force de sollicitation vers l'intérieur sur la bride de doigt (31) une fois que la bride de doigt (31) est insérée distalement depuis la face distale (61) de l'élément d'encliquetage (60),
dans laquelle
chaque saillie (70) fait saillie distalement depuis un élément d'encliquetage (60), formant ainsi une languette unique (40) avec l'élément d'encliquetage (60), la languette (40) étant configurée :
- pour fléchir élastiquement vers l'extérieur lors de l'insertion du dispositif de sécurité (103) dans l'enveloppe (50), afin de permettre l'insertion des brides de doigt (31) distalement depuis la face distale (61) de l'élément d'encliquetage (60) ;
**caractérisée en ce que** la languette (40) est configurée
- pour exercer une force de sollicitation vers l'intérieur sur la bride de doigt (31) lorsque la bride de doigt (31) est située distalement par rapport à la face distale (61) de l'élément d'encliquetage (60),
moyennant quoi la saillie (70) est configurée pour serrer le dispositif de sécurité (103) contre l'enveloppe (50) sensiblement orthogonalement à l'axe (105),
un espace entre la bride de doigt (31) et l'enveloppe (50) étant supprimé, de sorte que le dispositif de sécurité (103) soit empêché de bouger à l'intérieur de l'enveloppe (50).

2. Enveloppe selon la revendication 1, **caractérisée en ce que** l'enveloppe (50) est réalisée en une seule pièce.

3. Enveloppe selon la revendication 1 ou 2, **caractérisée en ce que** la saillie (70) a une face de maintien (71) configurée pour pousser la bride de doigt (31) vers l'intérieur, la face de maintien (71) s'étendant le long d'une direction sécante à la face distale (61) de l'élément d'encliquetage.

4. Enveloppe selon l'une quelconque des revendications 1 à 3, **caractérisée en ce que** l'élément d'encliquetage (60) comprend en outre une face proximale inclinée (62) pour faciliter l'insertion du dispositif de sécurité (103) dans l'enveloppe (50).

5. Enveloppe selon l'une quelconque des revendications 1 à 4, **caractérisée en ce que** la saillie (70) a une face proximale inclinée (72) pour faciliter l'insertion du dispositif de sécurité (103) dans l'enveloppe (50).

6. Enveloppe selon l'une quelconque des revendications 1 à 5, **caractérisée en ce qu'**une paroi (54) du boîtier (52) comprend une fente (41) qui a sensiblement la forme de U ayant deux pattes (42) s'étendant longitudinalement et une base (43) orthogonale à l'axe d'enveloppe (105), la zone de la paroi de boîtier (52) située à l'intérieur de la fente formant ladite languette (40).

7. Enveloppe selon l'une quelconque des revendications 1 à 6, **caractérisée en ce qu'**elle comprend au moins deux saillies (70), chacune étant agencée sur une paroi latérale (54) du boîtier (52), les saillies (70) étant opposées par rapport à l'axe (105) le long d'une direction latérale (X).

8. Enveloppe selon l'une quelconque des revendications 1 à 7, **caractérisée en ce qu'**elle comprend une saillie (70) sur chacune des parois latérales opposées (54) du boîtier (52).

9. Enveloppe selon l'une des revendications 1 à 8, **caractérisée en ce que** le boîtier (52) forme une partie élargie de l'enveloppe (50) par rapport au logement (51), ladite partie élargie s'étendant dans une direction latérale (X).

10. Enveloppe selon l'une quelconque des revendications 1 à 9, **caractérisée en ce qu'**elle comprend en outre une extrémité distale (57) munie d'un compartiment interne (58) configuré pour recevoir un dispositif de retrait de capuchon.

11. Enveloppe selon l'une des revendications 1 à 10, comprenant en outre au moins quatre nervures (80) à l'intérieur du logement (51), les nervures (80) étant configurées pour centrer le dispositif de sécurité (103) dans l'enveloppe (50).

12. Système de sécurité (102) pour une seringue (10), comprenant :
- un dispositif de sécurité (103) ayant un corps (20) configuré pour recevoir la seringue (10), et un protecteur (30) mobile par rapport au corps (20) et capable de couvrir une aiguille de seringue (14), le protecteur (30) ayant des brides de doigt (31) qui s'étendent latéralement de manière adjacente à une extrémité proximale du protecteur (30) ;
- une enveloppe (50) selon l'une quelconque des revendications précédentes, le dispositif de sécurité (103) étant monté dans l'enveloppe (50), les brides de doigt (31) du protecteur (30) de dispositif de sécurité étant maintenues par rapport à l'enveloppe (50) par le moyen d'encliquetage (60) et la saillie (70).

13. Système de seringue (100) comprenant un système de sécurité (102) selon la revendication 12 et une seringue (10) insérée dans le corps (20) du dispositif de sécurité (103), la seringue (10) comprenant une aiguille (14).

14. Système de seringue selon la revendication 13, **caractérisé en ce que** l'enveloppe (50) a une longueur telle que, dans la position montée, l'aiguille (14) dépasse l'extrémité distale (57) de l'enveloppe (50) d'une longueur prédéterminée.
